Europäisches Patentamt

European Patent Office

Office européen des brevets

(11) Publication number: **0 281 374 B1**

# EUROPEAN PATENT SPECIFICATION

(45) Date of publication of patent specification: **01.06.94**  (51) Int. Cl.5: **C12N 5/00, A01G 7/00**

(21) Application number: **88301804.6**

(22) Date of filing: **02.03.88**

(54) **Method of multiplicating plant seedlings.**

(30) Priority: **06.03.87 JP 50215/87**
**03.07.87 JP 165196/87**

(43) Date of publication of application:
**07.09.88 Bulletin 88/36**

(45) Publication of the grant of the patent:
**01.06.94 Bulletin 94/22**

(84) Designated Contracting States:
**BE DE FR GB IT LU NL**

(56) References cited:
EP-A- 0 133 978
EP-A- 0 137 504
EP-A- 0 142 987
US-A- 4 338 745

PLANTA, vol. 162, no. 1, 1984, pages 33-39,
Berlin; I. HAUSSER et al.

PLANT, CELL AND ENVIRONMENT, vol. 7, no.
6, 1984, pages 371-380, Oxford,GB; P. DIETER

(73) Proprietor: **MITSUI PETROCHEMICAL INDUS-**
**TRIES, LTD.**
**2-5, Kasumigaseki 3-chome**
**Chiyoda-ku**
**Tokyo 100(JP)**

(72) Inventor: **Tanimoto, Shizufumi**
**2-6, Misono 1-chome**
**Chiyoda-ku**
**Tokyo 100(JP)**

(74) Representative: **Myerscough, Philip Boyd et al**
**J.A.KEMP & CO.**
**14, South Square**
**Gray's Inn**
**London WC1R 5LX (GB)**

**Description**

The present invention concerns a method of multiplicating seedlings by applying tissue culture to plants.

Vegetables such as cabbages, tomatoes and cucumbers as well as rices have been utilized for foods. While on the other hand, horticultural plants such as tulips, bluebottle and rudbeckia have been favored as ornamental plants. These plants have been multiplicated so far by, for example, means of seedlings or division of bulbs or tubers. However, such multiplication methods not only require large areas and much labour, but also involve problems of the reduction of seedling growing rate or degradation in the quality of flowers due to the spread of virus diseases in recent years. With the aim of overcoming these problems and increasing the multiplication efficiency, methods utilizing the technique of plant tissue culture have been reported in recent years (for example, refer to Japanese Patent Laid-Open No. Sho 55-14734). Multiplication by the tissue culture technique has been attained by way of differentiation of adventitious bud, adventitious embryo or bulb, from cultured tissue pieces and cultured cells and it has been considered that the differentiation is controlled by the concentration ratio between cytokinin and auxin as plant hormones (for example, refer to Annals of Botany vol. 45, 321 - 327, 1980). However, there are many plant groups when undergo no differentiation only by the use of plant hormones or the frequency of differentiation therein, if it occurs, is extremely low. There has been a need for the establishment of a more direct and effective method of inducing differentiation.

We have studied a method of more efficiently multiplicating seedlings of plants as compared with the usual case based on the recognition that there are various problems in the method of tissue culture for plants as described above.

According to the invention there is provided a method of multiplicating plant seedlings by tissue culture characterised by effecting tissue culture after introducing an aqueous solution containing calmodulin and/or calcium directly into the cells of tissue pieces or cultured cells of the plants, said solution containing from 3 $\mu$g/ml to 1 mg/ml of calmodulin and/or from 100 $\mu$M to 30 mM of calcium.

The plants that can be used in the method of the present invention are not particularly restricted and the method can be applied to all kinds of plants.

Plants to which the method according to the invention can be applied include, for example, Papaveraceae, Solanaceae, Umbelliferae, Rosaceae, Liliaceae, Compositae, Geraniaceae, Cucurbitaceae and Gramineae. Specific examples of such plants can further include those described in "Ground for the plants System Classification" edited by Yamagishi, published from Kokuryukan in 1974. More specifically, there can be mentioned those plants belonging to Solanaceae such as egg plant, tomato, potato, sweet potato and basil, plants belonging to Papaveraceae such as poppy, rape, cabbage, radish, Chinese cabbage, plants belonging to Umbelliferae such as carrot, Japanese parsley and parsley, plants belonging to Rosaceae such as rose, strawberry, soybean and cherry, plants belonging to Lilaceae except for Lilium such as onions and tulips, plants belonging to Compositae such as chrysanthemum, bluebottle and sunflower, plants belonging to Geraniaceae such as pelargonium, geranium and flax, plants belonging to Cucurbitaceae such as cucumber and pumpkin, plants belonging to Gramineae such as rice and corn. Among these plants relevant to the present invention, preferred plants are, specifically, tomato, tobacco, egg plant, trenia, carrot, cabbage, onion, soybean, basil, bluebottle, rudbeckia, carnation, trumpet lily, tulip, asparagus, flax, cucumber and rice.

In the present invention, the tissue culture of plants is conducted by using tissue pieces or cultured cells of the plant. The tissue culture pieces can include, specifically, tissue pieces of plants prepared by slicing cotyledon, hypocotyl, shoot apex, stalk, leaf, scale, root or other tissue. These tissue pieces are used usually after sterilization with sodium hyperchlorite or ethanol. However, in the case of using aseptically cultured plant, the above-mentioned sterilized procedure is not required. Further, in the case of multiplicating seedlings of plants with no diseases and virus, tissues near the apical point, tissue pieces of the plants obtained from the tissue near the apical point can be used as the culture material. The cultured cells that can be used in the tissue culture of the plants in the present invention are undifferentiated amorphous cells including callus tissue obtained by applying tissue culture to the tissue pieces by the known method.

In the method of applying tissue culture according to the present invention, tissue culture is applied after introducing calmodulin and/or calcium into the cells of the tissue pieces or the culture cells.

Calmodulin in the present invention is a sort of protein such as $Ca^{2+}$-ATPase of erythrocyte membrane or phosphodiesterase of brain having activating function, the mobility of which is reduced under the presence of calcium upon conducting electrophoresis. Calmodulin can be isolated and purified, for example, from bulbs of trumpet lily, the cerebrum of cattle or seminal vesicle of rat.

Specific embodiments of the method of the present invention will now be described ;-

2

(1) Introducing calmodulin alone into the cells of tissue pieces or cultured cells of plants.

Calmodulin can be introduced for example, by the following methods. The calmodulin introducing treatment is carried out by placing tissue culture pieces or cultured cells between electrodes and, after adding a solution containing calmodulin thereto, electrical pulses at an electrical field intensity of from 30 V/cm to 2.5 KV/cm of electrical field intensity are applied for 30 $\mu$sec to 1 msec. In this case, calmodulin in the solution is at a concentration from 3 $\mu$g/ml to 1 mg/ml. In addition to the electrical injection process as described above, there can be mentioned various ways such as a method of injecting a solution containing calmodulin to the inside of the cells by using a micropipet made of glass under microscopic observation, a method of irradiating laser beam pulses to the cells placed in a calmodulin-containing solution to punctuate fine pores of submicron meter diameter to the cells and introducing calmodulin through the holes to the inside of cells by applying a method of transporting the material to the inside of the living cells as proposed by Japanese Patent Publication No. Sho 62-7838, or a method of introducing macro molecules to the inside of plant cells by using tungsten billets reported recently. The amount of calmodulin introduced to the inside of the cells by the methods described above can be determined by labelling the calmodulin with a fluorescent dye and measuring the fluorescence intensity after introduction to the inside of the cells and the amount is within a range usually from 1 $\mu$g/ml to 300 $\mu$g/ml and, preferably, from 10 $\mu$g/ml to 100 $\mu$g/ml.

In the method of applying tissue culture after introducing calmodulin to the inside of the cells of tissue pieces or cultured cells of plants according to the present invention, although tissue culture may be conducted by using the culture medium described later after the introduction of calmodulin, tissue culture applied by using a culture medium containing calcium ions usually by greater than 1 mM is more preferred since the differentiation is further promoted for the adventitious buds, adventitious embryo and bulbs of plants. In this case, it is desirable to apply tissue culture by using a culture medium containing calcium ionophores such as A23187 as proposed in EP-A-276575 published after the priority date of the present application within a range usually from $10^{-8}$ to $10^{-4}$M, preferably, from $10^{-7}$ to $10^{-5}$M, since the differentiation is remarkable. Furthermore, it is also preferable in the present invention to apply further tissue culture by introducing calmodulin after the tissue culture by using a culture medium containing calcium ionophers such as A23187 within a range from $10^{-8}$ to $10^{-4}$M since the differentiation is further promoted.

(2) Introducing calcium alone into the cells of tissue pieces or cultured cells of plants.

The calcium introduction method according to the present invention can be conducted in the same manner as in the case of introducing calmodulin. For instance, in the case of electrical injection, the tissue pieces of cultured cells are put between electrodes, calcium-containing solution is added and, thereafter, pulses are applied to introduce calcium to the inside of the cells.

The calcium concentration in the calcium-containing solution in this case, is from 100 $\mu$M to 30 mM. The calcium-containing solution can include, specifically, those aqueous solutions containing dissolved therein calcium compounds such as calcium chloride, calcium nitrate and calcium carbonate. In the present invention, the amount of calcium introduced to the inside of the cells by the above-mentioned method, when expressed in the same manner as for calmodulin, is within a range usually from $10^{-8}$M to $10^{-5}$M and, preferably, from $10^{-6}$M to $10^{-5}$M.

(3) Introducing both calmodulin and calcium into the cells of tissue pieces or cultured cells of plants.

This method is particularly preferred since the differentiation for the adventitious buds, adventitious embryos of plants is remarkably promoted as compared with the methods (1) and (2) above. The calmodulin and calcium can be introduced into the cells by the same procedures as in the case of (1) and (2) mentioned above and introduction can be attained, for instance, by placing the tissue pieces or cultured cells of plants between electrodes and, after adding a calcium-containing solution containing calmodulin, applying pulses at the same electrical field intensity as above in the same manners. In this case, the concentrations for the calmodulin and calcium ions in the solution are within the same ranges as those in (1) and (2) above. Further, the amounts of calmodulin and calcium ions introduced to the inside of the cells are also within the range of (1) and (2) described above.

Upon introducing calmodulin and calcium to the inside of cells in the present invention, it is usually desirable to employ a method of simultaneously introducing calcium ions and calmodulin joined together using a calcium-containng solution containing calmodulin as described above, but it is also possible to separately introduce calmodulin and calcium as required to the inside of the cells by the method as described above, followed by applying tissue culture.

Culture medium used for the tissue culture applied after the introduction of calmodulin and/or calcium can include, for example, those culture media as specifically described later.

The culture medium used in the present invention is a culture medium containing inorganic ingredients and carbon source as the essential ingredients, to which plant hormones and vitamins and, as required, amino acids are added. The inorganic ingredients for the culture medium can include those inorganic salts including elements such as nitrogen, phosphorus, potassium, sodium, calcium, magnesium, sulfur, iron, manganese, zinc, boron, molybdenum, chlorine, iodine and cobalts. Specifically, there can be mentioned those compounds such as potassium nitrate, sodium nitrate, ammonium nitrate, ammonium chloride, potassium chloride, calcium chloride, potassium monohydrogen phosphate, sodium dihydrogen phosphate, magnesium sulfate, magnesium chloride, sodium sulfate, ferrous sulfate, ferric sulfate, manganese sulfate, copper sulfate, sodium molybdate, molybdenum trioxide, potassium iodide, zinc sulfate, boric acid and cobalt chloride.

The carbon source for the culture medium can include, for example, carbohydrates and derivatives thereof such as sucrose, organic acids such as fatty acids and primary alcohols such as ethanol.

Plant hormones for the culture medium can include, for example, auxins such as naphthalene acetic acid (NAA), indole acetic acid (IAA), p-chlorophenoxyacetic acid, 2,4-dichlorophenoxy acetic acid (2,4-D), indole butyric acid (IBA) and derivatives thereof, and cytokinins such as benzyl adenine (BA), kinetin and zeatin.

Vitamins for the culture medium can include, for example, biotine, thiamine (vitamin B1), pyridoxine (vitamin B6), pyridoxal, pyridoxamine, calcium pantotate, ascorbic acid (vitamin C), inositol, nicotinic acid, nicotinic amide and riboflavine (vitamin B2).

The amino acid for the culture medium can include, for example, glycine, alanine, glutamic acid, cystein, phenyl alanine and lysine.

The culture medium used in the method of the present invention desirably incorporates from 0.1 $\mu$M to 100 mM of the inorganic ingredient, from 1 g/l to 100 g/l of the carbon source, from 0.01 mg/l to 10 mg/l of the plant hormones, from 0.1 mg/l to 150 mg/l of the vitamins and from 0 to 1000 mg/l of the amino acids.

The culture medium used for the tissue culture according to the present invention can include specifically those known culture media used for the tissue culture, for example, Murashige & Skoog culture medium ('62), Linsmaier & Skoog culture medium (RM-1965), White culture medium ('63), Gamborg B-5 culture medium, Mitsui M-9 culture medium, Nitch & Nitch culture medium, etc., being, incorporated as required with the carbon source and plant hormone as described above and, further, vitamins and amino acids as described above. Among them, those culture media prepared by using Nitch & Nitch, Linsmaier & Skoog or Marushige-Skoog media are preferred. In the present invention, it is also possible to use such culture medium prepared by adding calcium ionophore, cyclic AMP and polyamine as proposed in EP-A-276575 mentioned above. The compositions of the known culture media mentioned above are described, for example, in "New Plant Tissue Culture" p386 - p391, written by Takeuchi, Nakajima, Furuya, and published from Asakura Shoten in 1979.

The culture medium usable in the present invention is a liquid culture medium or solid culture medium usually containing from 0.1 to 2 % of gelling agent such as agar or gellite.

In the present invention, the tissue pieces or cultured cells of plants as described above can be applied with tissue culture by using a liquid culture medium aerated with oxygen-containing gas in the same manner as described in Japanese Patent Application No. Sho 60-128348.

According to the method of the present invention, it is possible to obtain a great amount of adventitious buds, adventitious embryos or bulblets (small bulbs) at a high efficiency from tissue pieces or cultured cells of plants. Referring more in this regard, adventitious buds obtained by the method according to the present invention can be rooted into plant bodies, which are then sliced into tissue pieces (bulblets are also sliced) and subjected further to the tissue culture by the culture method as described above according to the present invention thereby enabling to multiplicate seedlings in a great amount. Further, the plants obtained by the present invention can be grown into intact plant bodies of stable nature by usual cultivation.

By the use of the method according to the present invention, in which tissue culture is applied after introducing calmodulin and/or calcium into the cells of tissue pieces of cultured cells of plants, since the differentiation of adventitious buds, adventitious embryoes and bulbs of plants is remarkably promoted, a great amount of seedlings can be obtained. Accordingly, by the method of the present invention, plant bodies of high quality can be cultured in a great amount at a better efficiency as compared with the conventional method from the tissue pieces or cultured cells of plants. Furthermore, by the use of the method according to the present invention, the diameter of stalks of the differentiated adventitious buds can be increased to obtain satisfactory seedlings. The method is a novel tissue culture method based on the novel finding according to the present invention.

The nature and the advantageous effect of the present invention will now be described by reference to Examples.

4

Example 1

After sterilizing scales of trumpet lily bulbs with 70 % ethanol and an aqueous solution of sodium hyperchlorite (effective chlorine amount 1 %) and cutting into about 2 mm width, they were placed between electrodes, to which were added 3 mM solution of calcium chloride containing 100 $\mu$g/ml of calmodulin isolated and purified from the bulbs of trumpet lily and pulses of 500 V/cm of electric field intensity for 200 $\mu$sec were three times applied. Then, aseptic Murashige and Skoog solid culture medium (1962) (gellite concentration, 0.2%) at pH 6.0 containing 4 % sucrose, 0.01 mg/liter of naphthalene acetic acid and 0.02 mg/liter of benzyl adenine was prepared. 10 pieces of the scales of the trumpet lily bulbs described above were added to the medium and cultured at 25°C in a bright place for 3 weeks, the results as shown in Table 1 were obtained as the number of bulbs formed per slice. The number of bulbs obtained by differentiation was increased in any of the treated specimens as compared with those in Comparative Example 1.

Comparative Example 1

Slices of trumpet lily scales were cultured in the same procedures as in Example 1 excepting for using distilled water instead of the calcium chloride solution containing calmodulin in Example 1.

Examples 2 - 3

Tissue culture was applied to trumpet lily in the same procedures as in Example 1 excepting for using callus cells of trumpet lily instead of slices of trumpet lily leaves as the material in Example 1, to obtain the results as shown in Table 1. The number of differentiated bulbs was increased in any of the treated specimens as compared with those in Comparative Examples 2 - 3.

Comparative Examples 2 - 3

Slices of trumpet lily leaves and callus cells of trumpet lily were cultured in the same procedures as in Examples 2 - 3 excepting for using distilled water instead of the calcium chloride solution containing calmodulin in Example 2 - 3 and results are shown in Table 1.

Examples 4 - 5

Tissue culture was applied to callus cells of trumpet lily in the same procedures as in Example 3 excepting for using calmodulin isolated and purified from cerebrum of cattle or seminal vesicle of rat as calmodulin introduced in that example. The results are shown in Table 1.

5

Table 1

| | Material | Treatment | Number of bulbs formed/slice |
|---|---|---|---|
| Example 1 | Slice of trumpet lily scales | Treated with introduction of calmodulin and calcium | 4.7 |
| Comparative Example 1 | " | Treated with treated water | 2.1 |
| Example 2 | slice of trumpet lily leaves | Treated with introduction of calmodulin and calcium | 12.8 |
| Example 3 | Callus cells of trumpet lily | " | 21.5 |
| Comparative Example 2 | Slice of trumpet lily leaves | Treated with distilled water | 1.4 |
| " 3 | Callus cells of trumpet lily | " | 2.3 |
| Example 4 | " | Treated with introduction of calmodulin and calcium | 17.6 |
| " 5 | " | " | 18.5 |

Examples 6 - 14

Tissue culture was applied in the same procedures as in Example 1 excepting for using slices of tomato leaves, callus cells of tomato, slices of egg plant leaves, callus cells of egg plant, slices of tobacco leaves, slices of tobacco leaves, slices of trenia stalks, slices of carnation stalks, slices of cabbage hypocotyl and slices of onion scales as material in Example 1. The results are shown in Table 2. The number of adventitious buds obtained by differentiation was increased in any of the treated specimens as compared with those in Comparative Examples 4 - 12.

Comparative Examples 4 - 12

Tissue culture was applied to slices of tomato leaves, callus cells of tomato, slices of egg plant leaves, callus cells of egg plant, slices of tobacco leaves, slices of trenia stalks, slices of carnation stalks, slices of cabbage hypocotyl and slices of onion scales except for using distilled water instead of the calcium chloride solution containing calmodulin in the examples. The results are shown in Table 2.

Table 2

| | | Material | Treatment | Number of adventitious buds formed/slice |
|---|---|---|---|---|
| Example | 6 | Slice of totato leaves | Treated with introduction of calmodulin and calcium | 4.2 |
| " | 7 | Callus cells of tomato | " | 8.5 |
| " | 8 | slice of egg plant leaves | " | 4.0 |
| " | 9 | Callus cells of egg plant | " | 6.4 |
| " | 10 | Slice of tobacco leaves | " | 7.0 |
| " | 11 | Slice of trenia stalks | " | 32.4 |
| " | 12 | Slice of carnation stalks | " | 0.7 |
| " | 13 | Slice of cabbage hypocotyl | " | 3.4 |
| " | 14 | Slce of onion scales | " | 9.8 |
| Comparative Examples | 4 | Slice of tomato leaves | Treatment with distilled water | 2.8 |
| " | 5 | Callus cells of tomato | " | 3.8 |
| " | 6 | Slice of egg plant leaves | " | 2.4 |
| " | 7 | Callus cells of egg plant | " | 3.2 |
| " | 8 | Slice of tobacco leaves | " | 1.6 |
| " | 9 | Slice of trenia stalks | " | 8.4 |
| " | 10 | Slice of carnation stalks | " | 0 |
| " | 11 | Slice of cabbage hypocotyl | " | 0.2 |
| " | 12 | Slice of onion scales | " | 4.6 |

Examples 15 - 20

Tissue culture was applied in the same procedures as in Example 1 excepting for using slices of basil leaves, slices of bluebottle cotyledon, slices of rudbekia leaves, slices of tulip scales, slices of flax stalks and slices of asparagus stalks, in that example and the results are shown in Table 3.

Comparative Examples 13 - 18

Tissue culture was applied to slices of basil leaves, slices of bluebottle cotyledon, slices of rudbekia leaves, slices of tulip scales, slices of flax stalks and slices of asparagus stalks excepting for distilled water instead of the calcium chloride solution containing calmodulin in Examples 15 - 20 and the results are shown in Table 3.

Table 3

| | | Material | Treatment | Number of adventitious buds formed/slice |
|---|---|---|---|---|
| Example | 15 | Slice of basil leaves | Treated with introduction of calmodulin *and Calcium* | 8 . 4 |
| " | 16 | Slice of bluebottle cotyledon | " | 7 . 6 |
| " | 17 | Slice of rudbeckia | " | 5 . 0 |
| " | 18 | Slice of tulip scales | " | 8 . 6 |
| " | 19 | Slice of flax stalks | " | 1 3 . 8 |
| " | 20 | Slice of asparagus stalks | " | 3 . 8 |
| Comparative Example | 13 | Slice of basil leaves | Treated with distilled water | 3 . 2 |
| " | 14 | Slice of bluebottle cotyledon | " | 2 . 2 |
| " | 15 | Slice of rudgeckia leaves | " | 2 . 0 |
| " | 16 | Slice of tulip scales | " | 3 . 8 |
| " | 17 | Slice of flax stalks | " | 6 . 0 |
| " | 18 | Slice of asparagus stalks | " | 0 . 6 |

Examples 21 - 28 and Comparative Example 19

After cutting the scales of sterilized trumpet lily bulbs used in Example 1 to about 2 mm width, they were placed between electrodes, to which was added a solution containing calmodulin isolated and purified from the bulbs of trumpet lily at a concentration shown in Table 4 and calcium chloride at a concentration also shown in Table 4. Then, after applying electrical pulses under the same conditions as those in Example 1, the slices of trumpet lily scales were cultured in the same procedures in Example 1. The results are shown in Table 4.

Calmodulin introduced into the cells by the introduction treatment was about 33 μg/ml and the $Ca^{2+}$ concentration in the cells was increased from $10^{-7}$ M to $6\times10^{-5}$ M in Example 27.

Table 4

| | Concentration in the solution | | Number of bulbs formed/slice |
|---|---|---|---|
| | $Ca^{2+}$ (mM) | Calmodulin (μg/mℓ) | |
| Comparative Example 19 | 0 | 0 | 2.8 |
| Example 21 | 0 | 100 | 3.5 |
| " 22 | 0.1 | 100 | 7.5 |
| " 23 | 0.3 | 100 | 13.0 |
| " 24 | 1 | 100 | 14.0 |
| " 25 | 3 | 0 | 9.5 |
| " 26 | 3 | 10 | 10.5 |
| " 27 | 3 | 100 | 21.5 |
| " 28 | 10 | 100 | 11.5 |

Examples 29 - 37

Tissue culture was applied in the same procedures as in Example 1 using solutions containing calcium chloride at concentrations shown in Table 5 in Examples 10, 11 and 14. The results are shown in Table 5.

Examples 38 - 40

After culturing the tissue pieces or cultured cells for 3 to 7 days each in a medium containing $10^{-6}$ M of A23187 in Examples 3, 11 and 14, electrical pulses were applied to 100 $\mu$g/ml of calmodulin solution used, and tissue culture was further applied. The results are shown in Table 6.

Examples 41 - 43

After conducting calmodulin introduction in the same procedures as in Example 1 using 100 $\mu$g/ml of calmodulin in Examples 3, 11 and 14, tissue culture was applied using culture medium containing $10^{-6}$ M of A23187. The results are shown in Table 6.

EP 0 281 374 B1

Table 5

| | | Material | Concentration in the solution Ca$^{2+}$ ( m M ) | Number of adventitious buds formed/slice |
|---|---|---|---|---|
| Example | 2 9 | Slice of tobacco leaves | 1 | 2 . 6 |
| " | 3 0 | " | 3 | 6 . 2 |
| " | 3 1 | " | 1 0 | 4 . 0 |
| " | 3 2 | Slice of trenia stalks | 1 | 1 0 . 2 |
| " | 3 3 | " | 3 | 2 2 . 6 |
| " | 3 4 | " | 1 0 | 1 4 . 2 |
| " | 3 5 | Slice of onion scales | 1 | 4 . 8 |
| " | 3 6 | " | 3 | 8 . 0 |
| " | 3 7 | " | 1 0 | 5 . 2 |

12

Table 6

| Example | Material | Treatment | Number of bulbs formed/slice | Number of adventitious buds formed/slice |
|---|---|---|---|---|
| Example 38 | Callus cells of trumpet lily | Treated with introduction of calmodulin | | 26.8 |
| " 39 | Slice of trenia stalks | " | | 8.6 |
| " 40 | Slice of onion scales | " | 18.6 | |
| " 41 | Callus cells of trumpet lily | " | | 30.6 |
| " 42 | Slice of trenia stalks | " | | 10.2 |
| " 43 | Slice of onion scales | " | 22.0 | |

Examples 44 - 49

Tissue culture was applied in the same procedures as in Example 1 excepting for using slices of carrot hypocotyl, callus cells of soybean, callus cells of cucumber, callus cells of rice, callus cells of snapdragon, callus cells of asparagus as the material in that example and the results are shown in Table 7. The number of adventitious embryoes obtained by differentiation was increased in any of treated specimens as compared with those in Comparative Examples 20 - 25.

Comparative Examples 20 -25

Slices of carrot hypocotyl, callus cells of soybean, callus cells of cucumber, callus cells of rice, callus cells of snapdragon, callus cells of asparagus were cultured in the same procedures as in Example 1 excepting for using distilled water instead of using the calcium chloride solution containing calmodulin in Examples 44 - 49.

Examples 50 - 52

Slices of asparagus callus stalks were applied with tissue culture in the same manner as in Example 1 using solutions containing calmodulin in Example 20 at concentrations shown in Table 8 and calcium chloride at concentrations also shown in Table 8 and the results are shown in Table 8. The number of adventitious buds obtained by the differentiation was increased in any of the treated specimens as compared with that in Comparative Example 26, and the diameter of the stalks was also increased as that in Comparative Example 26.

Comparative Example 26

Slice of asparagus stalks were cultured in the same procedures as in Examples 50 - 52 excepting for using distilled water instead of the calcium chloride solution containing calmodulin in these examples.

Table 7

| | | Material | Treatment | Number of adventitious embryo formed/external slice (0.1 g) |
|---|---|---|---|---|
| Example | 44 | Slice of carroy hypocotyl | Treated with introduction calmodulin | 0 . 4 |
| " | 45 | Callus cells of soybean | " | 1 . 0 |
| " | 46 | Callus cells of cucumber | " | 2 . 4 |
| " | 47 | Callus cells of rice | " | 2 . 2 |
| " | 48 | Callus cells of snapdragon | " | 3 . 6 |
| " | 49 | Callus cells of asparagus | " | 4 . 2 |
| Comparative Example | 20 | Slice of carrot hypocotyl | Treated with distilled water | 0 |
| " | 21 | callus cells of soybean | " | 0 |
| " | 22 | Callus cells of cucumber | " | 0 |
| " | 23 | Callus cells of rice | | 0 . 8 |
| " | 24 | Callus cells of snapdragon | | 0 . 6 |
| " | 25 | Callus cells of asparagus | | 2 . 4 |

EP 0 281 374 B1

Table 8

| | Concentration in solution | | Number of adventitious buds formed/slice | Adventitious buds diameter (mm) |
| | Ca²⁺ (mM) | Calmodulin (μg/mℓ) | | |
|---|---|---|---|---|
| Example 26 | 0 | 0 | 0.5 | 1.1 |
| Comparative Example 50 | 0 | 100 | 2.4 | 2.3 |
| " 51 | 1 | 100 | 3.2 | 2.6 |
| " 52 | 3 | 100 | 4.4 | 4.2 |

Example 53

Tissue culture was applied in the same procedures as in Example 1 except for using callus cells induced from the slices of stalks and slices of leaves of miniature rose (variety : Marie Antoinette) in that

EP 0 281 374 B1

example. The results are shown in Table 9. The number of the treated adventitious buds was increased as compared with that in Comparative Example 27.

Comparative Example 27

Callus cells of miniature rose were cultured in the same procedures as in Example 53 except for using distilled water instead of the calcium chloride solution containing calmodulin in Example 53.

## Table 9

|  | Material | Treatment | Number of adventitious buds formed/callus slice (0.1 g) |
|---|---|---|---|
| Example 53 | callus cells of miniature rose | Treated with introduction calmodulin | 5.2 |
| Comparative Example 27 | " | Treated with distilled water | 2.4 |

## Claims

1. A method of multiplicating plant seedlings by tissue culture characterised by effecting tissue culture after introducing an aqueous solution containing calmodulin and/or calcium directly into the cells of tissue pieces or cultured cells of the plants, said solution containing from 3 $\mu$g/ml to 1 mg/ml of calmodulin and/or from 100 $\mu$M to 30 mM of calcium.

2. A method according to claim 1, wherein calmodulin and/or calcium is introduced into the cells by injecting the solution by applying electrical pulses thereto.

3. A method according to claim 1, wherein calmodulin and/or calcium is introduced into the cells by injecting the solution using a micropipet.

4. A method according to claim 1, wherein calmodulin and/or calcium is introduced into the cells by applying laser beam irradiation to make fine pores in the cells and introducing the solution through the fine pores.

5. A method according to any one of claims 1 to 4 wherein there is used calmodulin obtained from bulbs of trumpet lily, cerebrum of cattle or seminal vesicle of rat by isolation and purification.

6. A method according to any one of the preceding claims wherein tissue culture is effected in a culture medium containing from $10^{-8}$ to $10^{-4}$ mol of calcium ionophore.

7. A method according to any one of the preceding claims wherein tissue culture is effected in Murashige and Skoog medium.

## Patentansprüche

1. Verfahren zum Vermehren von Pflanzensetzlingen durch Züchten von Gewebe, dadurch gekennzeichnet daß man das Züchten des Gewebes durchführt, nachdem man eine wässerige, Calmodulin und/oder Kalzium enthaltende Lösung direkt in die Zellen von Gewebestücken oder in die gezüchteten Zellen der Pflanzen eingebracht hat, wobei die erwähnte Lösung 3$\mu$g/ml bis 1 mg/ml Calmodulin

17

und/oder 100 $\mu$Mol bis 30 mMol Kalzium enthält.

2. Verfahren nach Anspruch 1, wobei Calmodulin und/oder Kalzium durch Einspritzen der Lösung in die Zellen eingebracht wird, indem man elektrische Impulse auf sie anwendet.

3. Verfahren nach Anspruch 1, wobei Calmodulin und/oder Kalzium durch Einspritzen unter Verwendung einer Mikropipette in die Zellen eingebracht wird.

4. Verfahren nach Anspruch 1, wobei Calmodulin und/oder Kalzium dadurch in die Zellen einbringt, daß man eine Bestrahlung mit Laserstrahlen anwendet, um feine Poren in den Zellen zu erzeugen und die Lösung durch die feinen Poren einbringt.

5. Verfahren nach einem der Ansprüche 1 bis 4, wobei Calmodulin verwendet wird, das aus Knollen der Trompetenlilie, Rinderhirn oder Samenbläschen von Ratten durch Isolierung und Reinigung erhalten wurde.

6. Verfahren nach einem der vorausgehenden Ansprüche, wobei die Gewebezüchtung in einem Züchtungsmedium ausgeführt wird, das $10^{-8}$ bis $10^{-4}$ Mol einer Kalzium enthaltenden Verbindung (Calonophor) enthält.

7. Verfahren nach einem der vorausgehenden Ansprüche, wobei die Gewebezüchtung in einem Murashige- und Skoog-Medium durchgeführt wird.

**Revendications**

1. Procédé de multiplication de plants de plante par culture de tissu, caractérisé en ce que l'on effectue la culture de tissu après introduction d'une solution aqueuse contenant de la calmoduline et/ou du calcium, directement dans les cellules de morceaux de tissu ou des cellules cultivées des plantes, ladite solution contenant de 3 $\mu$g/ml à 1 mg/ml de calmoduline et/ou de 100 $\mu$M à 30 mM de calcium.

2. Procédé selon la revendication 1, dans lequel on introduit la calmoduline et/ou le calcium dans les cellules en injectant la solution par application d'impulsions électriques aux cellules.

3. Procédé selon la revendication 1, dans lequel on introduit la calmoduline et/ou le calcium dans les cellules en injectant la solution à l'aide d'une micropipette.

4. Procédé selon la revendication 1, dans lequel on introduit la calmoduline et/ou le calcium dans les cellules en exposant les cellules à un faisceau laser de façon à créer des pores fins dans les cellules et en introduisant la solution par les pores fins.

5. Procédé selon l'une quelconque des revendications 1 à 4, dans lequel on utilise de la calmoduline obtenue à partir de bulbes de lys (trumpet lily), de cerveau de bétail ou de vésicule séminale de rat, par isolement et purification.

6. Procédé selon l'une quelconque des revendications précédentes, dans lequel on effectue la culture de tissu dans un milieu de culture contenant de $10^{-8}$ à $10^{-4}$ mole d'ionophore calcium.

7. Procédé selon l'une quelconque des revendications précédentes, dans lequel on effectue la culture de tissu dans un milieu de Murashige et Skoog.